# EUROPEAN PATENT APPLICATION

(11) **EP 4 606 889 A1**
(43) Date of publication of application: **27.08.2025**
(21) Application number: 23880227.6
(22) Date of filing: 18.10.2023
(51) Int. Cl.: C12N 5/078

(54) **MEDIUM COMPOSITION FOR INDUCING DIFFERENTIATION FROM STEM CELL TO PLATELET PROGENITOR CELL, AND DIFFERENTIATION METHOD USING SAME**

(30) Priority: 18.10.2022 KR 20220134329
(71) Applicant: Sungkwang Medical Foundation, Seoul 06135 (KR); Cha University Industry-Academic Cooperation Foundation, Pocheon-si, Gyeonggi-do 11160 (KR)
(72) Inventor: KANG, Eun Ju, Seoul 04595 (KR); LEE, Yeon Mi, Gwangju-si, Gyeonggi-do 12787 (KR); KIM, Young Hee, Ansan-si, Gyeonggi-do 15596 (KR)
(74) Representative: Vossius & Partner Patentanwälte Rechtsanwälte mbB
(86) International application number: PCT/KR2023/016168
(87) International publication number: WO 2024/085650

(57) **Abstract**

Provided are a culture medium composition for inducing differentiation from stem cells to platelet precursor cells and a method of inducing differentiation from stem cells to platelet precursor cells in the presence of said culture medium composition. According to one aspect, the culture medium composition for inducing differentiation from stem cells to platelet precursor cells can enhance the efficiency of differentiation into platelet precursor cells and has the effect of differentiating into platelet precursor cells without genetic manipulation.

## Description

### Technical Field

The present disclosure relates to a culture medium composition for inducing differentiation of stem cells into platelet progenitor cells.

### Background Art

The treatment of blood-related diseases and surgical treatments require large quantities of blood cells. Among blood cells, platelets, which are essential cells for blood coagulation and hemostasis, are particularly important. Platelet preparations are administered to patients exhibiting bleeding tendencies due to massive bleeding during surgery or injury, or thrombocytopenia after chemotherapy, for the purpose of treating and preventing these symptoms. Currently, the production of platelet preparations relies on blood donations from healthy volunteers, but the existing blood supply system and blood transfusions are reaching their limits.

*In vivo,* megakaryocytes form pseudopodial formations called proplatelets and fragment their cytoplasm to release platelets. Megakaryocytes are thought to become polyploid by endomitosis until they release platelets. Endomitosis of megakaryocytes is a multipolar mitosis caused by abnormalities in nuclear division and cytoplasmic division, without cleavage furrow formation and spindle elongation, resulting in cells containing several lobulated nuclei. This repeated endomitosis induces polyploidization of megakaryocytes.

Recently, techniques for inducing differentiation of pluripotent stem cells, such as ESCs (embryonic stem cells) or iPSCs (induced pluripotent stem cells), *in vitro* to prepare blood cells such as platelets have also been studied. However, there is no differentiation technology that can mass-produce artificial platelets due to the low efficiency of differentiation induction into megakaryocytes. Therefore, there is a need for a technique that induces differentiation into megakaryocytes with high purity without gene introduction.

### Disclosure of Invention

### Technical Problem

An aspect is a culture medium composition for inducing differentiation of stem cells into platelet progenitor cells, the culture medium composition including: (i) a first culture medium composition for inducing differentiation of the stem cell into a hematopoietic progenitor cell or a hematopoietic stem cell, the first culture medium composition comprising at least one selected from the group consisting of CHIR99021, Bone Morphogenetic Protein 4 (BMP4), Vascular Endothelial Growth Factor (VEGF), Basic Fibroblast Growth Factor (bFGF), retinoic acid, SB-431542, polyvinyl alcohol (PVA), Stem Cell Factor (SCF), Interleukin 3 (IL3), valproic acid (VPA), and combinations thereof; (ii) a second culture medium composition for inducing differentiation of the hematopoietic progenitor cell or the hematopoietic stem cell into a megakaryocyte progenitor cell, the second culture medium composition comprising at least one selected from the group consisting of SCF, Thrombopoietin (TPO), PVA, bFGF, butyzamide, Interleukin 6 (IL6), UM729, Macrophage Colony-Stimulating Factor (M-CSF), and combinations thereof; and (iii) a third culture medium composition for inducing maturation of the megakaryocyte progenitor cell into a platelet progenitor cell, the third culture medium composition comprising at least one selected from the group consisting of SCF, TPO, Interleukin 6 (IL6), fasudil, butyzamide, bFGF, and combinations thereof.

Another aspect provides a method of inducing differentiation of a stem cell into a platelet progenitor cell, the method including: inducing differentiation of the stem cell into a hematopoietic progenitor cell or a hematopoietic stem cell in the presence of the first culture medium composition; inducing differentiation of the hematopoietic progenitor cell or the hematopoietic stem cell into a megakaryocyte progenitor cell in the presence of the second culture medium composition; and inducing maturation of the megakaryocyte progenitor cell into a platelet progenitor cell in the presence of the third culture medium composition.

### Solution of Problem

One aspect provides a culture medium composition for inducing differentiation of stem cells into platelet progenitor cells, including: (i) a first culture medium composition for inducing differentiation of the stem cell into a hematopoietic progenitor cell or a hematopoietic stem cell, the first culture medium composition comprising at least one selected from the group consisting of CHIR99021, Bone Morphogenetic Protein 4 (BMP4), Vascular Endothelial Growth Factor (VEGF), Basic Fibroblast Growth Factor (bFGF), retinoic acid, SB-431542, polyvinyl alcohol (PVA), Stem Cell Factor (SCF), Interleukin 3 (IL3), valproic acid (VPA), and combinations thereof; (ii) a second culture medium composition for inducing differentiation of the hematopoietic progenitor cell or the hematopoietic stem cell into a megakaryocyte progenitor cell, the second culture medium composition comprising at least one selected from the group consisting of SCF, Thrombopoietin (TPO), PVA, bFGF, butyzamide, Interleukin 6 (IL6), UM729, Macrophage Colony-Stimulating Factor (M-CSF), and combinations thereof; and (iii) a third culture medium composition for inducing maturation of the megakaryocyte progenitor cell into a platelet progenitor cell, the third culture medium composition comprising at least one selected from the group consisting of SCF, TPO, Interleukin 6 (IL6), fasudil, butyzamide, bFGF, and combinations thereof.

In the present specification, the term "megakaryocyte" may be used interchangeably with the term "platelet progenitor cell," and refers to a large (e.g., diameter ≥ 10 µm) polyploid hematopoietic cell that has a tendency to produce proplatelets and/or platelets. The megakaryocyte is characterized by being positive for the cell surface markers CD41a, CD42a, and CD42b, and in addition, may further express markers selected from the group consisting of CD9, CD61, CD62p, CD42c, CD42d, CD49f, CD51, CD110, CD123, CD131, and CD203c. One morphological feature of a mature megakaryocyte is the development of a large, multi-lobed nucleus. Although a mature megakaryocyte can cease to proliferate, it can continue to increase its DNA content through endomitosis, with a parallel increase in cell size.

The term "platelet" refers to a cell having a diameter of 1 µm to 3 µm that lacks a nucleus but contains RNA. A platelet can express CD41, CD42b, and CD61 on its cell surface. Platelets function primarily in hemostasis control by participating in blood coagulation, but have also been suggested to play a role in inflammation.

The term "proplatelet" refers to a cytoplasmic extension from a megakaryocyte or one that has just been released from a megakaryocyte. Proplatelets separate through cytoskeletal rearrangement to form individual platelets.

In the present disclosure, the term "CHIR99021" refers to a compound having the structure of Formula 1 below.

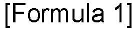

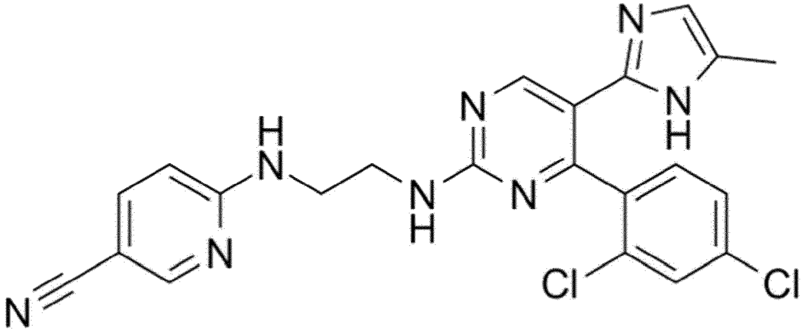

In the present disclosure, the term "SB-431542" refers to a compound having the structure of Formula 2 below.

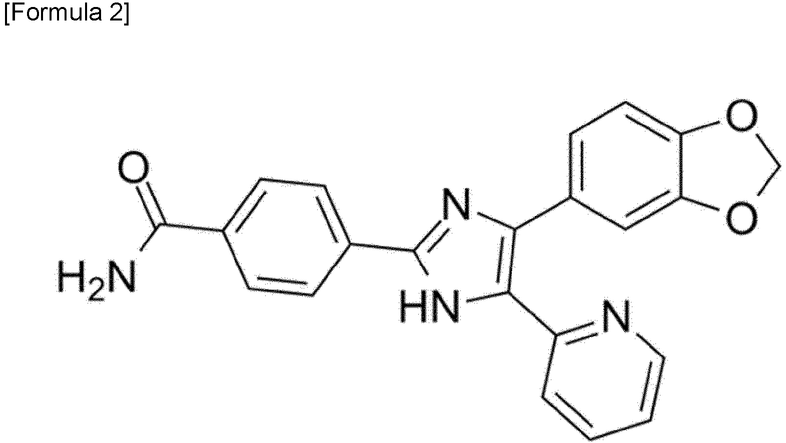

In the present disclosure, the term "UM729" refers to a compound having the structure of Formula 3 below.

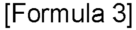

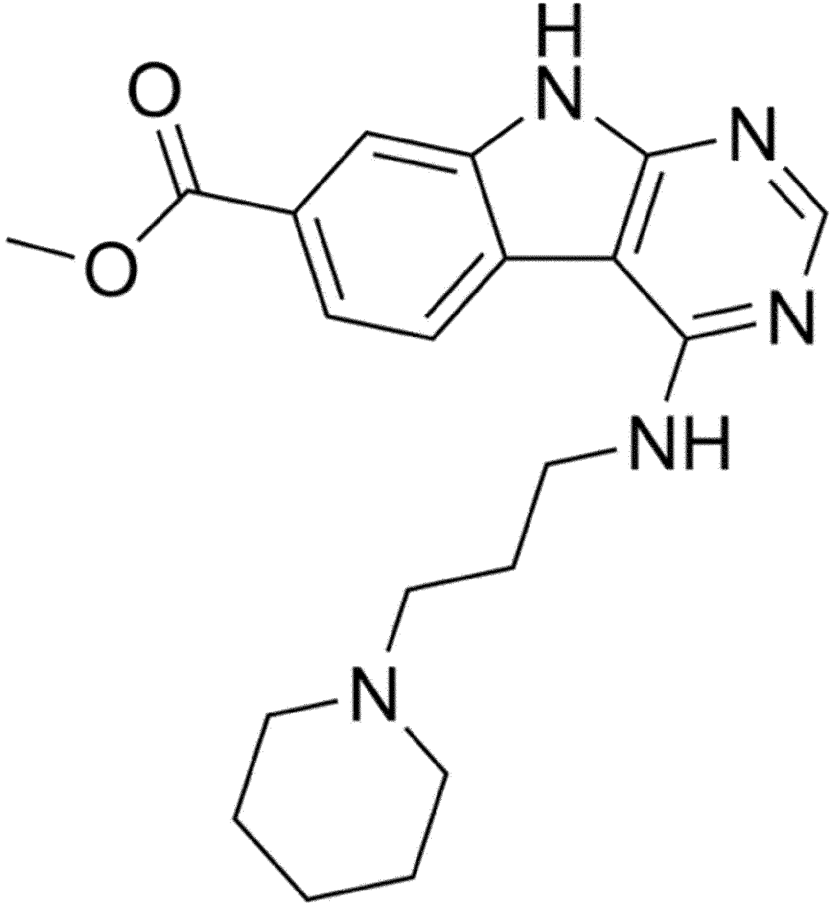

In the present disclosure, the term "KP-457" refers to a compound having the structure of Formula 4 below.

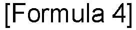

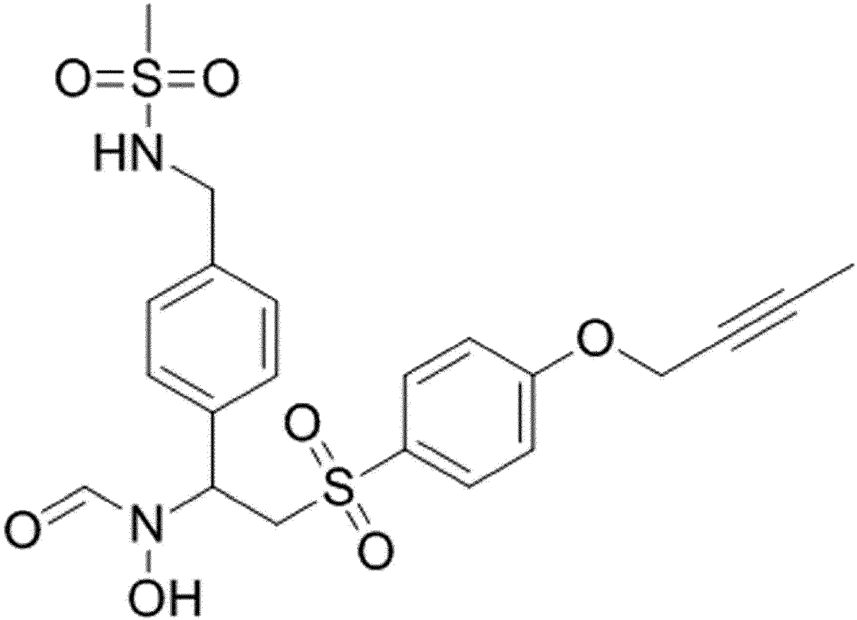

In the present disclosure, "medium" may vary depending on the intended use, but refers to an animal cell culture medium that basically contains inorganic salts, a carbon source, amino acids, bovine serum albumin (BSA), and cofactors, and may encompass all common media well known to those skilled in the art. Particularly preferably, the medium may include NaCl, KCI, and NaHCO₃ as inorganic salts; glucose, sodium pyruvate, and calcium lactate as carbon sources; essential amino acids (including glutamine) and non-essential amino acids as amino acids; and may further include other trace elements and buffers as cofactors.

As the medium, various commercially known animal cell culture media-for example, Dulbecco's Modified Eagle Medium (DMEM), Endothelial Differentiation Medium (EDM), Minimal Essential Medium (MEM), Basal Medium Eagle (BME), Roswell Park Memorial Institute 1640 (RPMI 1640), F-10, F-12, Alpha Minimal Essential Medium (α-MEM), Glasgow's Minimal Essential Medium (G-MEM), and Iscove's Modified Dulbecco's Medium (IMDM)-can be used as is, but are not limited thereto. In an embodiment, StemPro-34 SFM medium may be used.

In an embodiment, the concentration of CHIR99021 in the first culture medium composition may be in a range of 0.5 µM to 50 µM, 0.5 µM to 40 µM, 0.5 µM to 30 µM, 0.5 µM to 20 µM, 0.5 µM to 10 µM, 1 µM to 50 µM, 2 µM to 50 µM, 3 µM to 50 µM, 4 µM to 50 µM, 1 µM to 40 µM, 2 µM to 30 µM, 3 µM to 20 µM, or 4 µM to 10 µM.

In an embodiment, the concentration of BMP4 in the first culture medium composition may be in a range of 5 ng/ml to 500 ng/ml, 5 ng/ml to 400 ng/ml, 5 ng/ml to 300 ng/ml, 5 ng/ml to 200 ng/ml, 5 ng/ml to 100 ng/ml, 10 ng/ml to 500 ng/ml, 20 ng/ml to 500 ng/ml, 30 ng/ml to 500 ng/ml, 40 ng/ml to 500 ng/ml, 10 ng/ml to 400 ng/ml, 20 ng/ml to 300 ng/ml, 30 ng/ml to 200 ng/ml, or 40 ng/ml to 100 ng/ml.

In an embodiment, the concentration of VEGF in the first culture medium composition may be in a range of 5 ng/ml to 500 ng/ml, 5 ng/ml to 400 ng/ml, 5 ng/ml to 300 ng/ml, 5 ng/ml to 200 ng/ml, 5 ng/ml to 100 ng/ml, 10 ng/ml to 500 ng/ml, 20 ng/ml to 500 ng/ml, 30 ng/ml to 500 ng/ml, 40 ng/ml to 500 ng/ml, 10 ng/ml to 400 ng/ml, 20 ng/ml to 300 ng/ml, 30 ng/ml to 200 ng/ml, or 40 ng/ml to 100 ng/ml.

In an embodiment, the concentration of bFGF in the first culture medium composition may be in a range of 1 ng/ml to 1,000 ng/ml, 1 ng/ml to 900 ng/ml, 1 ng/ml to 800 ng/ml, 1 ng/ml to 700 ng/ml, 1 ng/ml to 600 ng/ml, 1 ng/ml to 500 ng/ml, 1 ng/ml to 400 ng/ml, 1 ng/ml to 300 ng/ml, 1 ng/ml to 200 ng/ml, 10 ng/ml to 1,000 ng/ml, 20 ng/ml to 1,000 ng/ml, 30 ng/ml to 1,000 ng/ml, 40 ng/ml to 1,000 ng/ml, 10 ng/ml to 900 ng/ml, 20 ng/ml to 800 ng/ml, 30 ng/ml to 700 ng/ml, 40 ng/ml to 600 ng/ml, 40 ng/ml to 500 ng/ml, 40 ng/ml to 400 ng/ml, 40 ng/ml to 300 ng/ml, 40 ng/ml to 200 ng/ml, 10 ng/ml to 200 ng/ml, or 10 ng/ml to 100 ng/ml.

In an embodiment, the concentration of retinoic acid in the first culture medium composition may be in a range of 0.1 µM to 10 µM, 0.1 µM to 8 µM, 0.1 µM to 6 µM, 0.1 µM to 4 µM, 0.1 µM to 2 µM, 0.3 µM to 10 µM, 0.5 µM to 10 µM, 0.7 µM to 10 µM, 0.9 µM to 10 µM, 0.3 µM to 8 µM, 0.5 µM to 6 µM, 0.7 µM to 4 µM, or 0.9 µM to 2 µM.

In an embodiment, the concentration of SB-431542 in the first culture medium composition may be in a range of 1 µM to 100 µM, 1 µM to 80 µM, 1 µM to 60 µM, 1 µM to 40 µM, 1 µM to 20 µM, 3 µM to 100 µM, 5 µM to 100 µM, 7 µM to 100 µM, 9 µM to 100 µM, 3 µM to 80 µM, 5 µM to 60 µM, 7 µM to 40 µM, or 9 µM to 20 µM.

In an embodiment, the concentration of PVA in the first culture medium composition may be in a range of 0.01 %(w/v) to 10 %(w/v), 0.01 %(w/v) to 5 %(w/v), 0.01 %(w/v) to 3 %(w/v), 0.01 %(w/v) to 2 %(w/v), 0.01 %(w/v) to 1 %(w/v), 0.01 %(w/v) to 0.8 %(w/v), 0.01 %(w/v) to 0.6 %(w/v), 0.01 %(w/v) to 0.4 %(w/v), 0.01 %(w/v) to 0.2 %(w/v), 0.01 %(w/v) to 0.1 %(w/v), 0.03 %(w/v) to 5 %(w/v), 0.05 %(w/v) to 3 % (w/v), 0.09 %(w/v) to 2 %(w/v), 0.1 %(w/v) to 1 %(w/v), 0.05 %(w/v) to 10 %(w/v), 0.1 %(w/v) to 10 %(w/v), 0.5 %(w/v) to 8 %(w/v), 0.7 %(w/v) to 7 %(w/v), 0.8 %(w/v) to 6 %(w/v), 0.9 %(w/v) to 5 %(w/v), or 0.9 %(w/v) to 2 %(w/v).

In an embodiment, the concentration of SCF in the first culture medium composition may be in a range of 2 ng/ml to 200 ng/ml, 2 ng/ml to 150 ng/ml, 2 ng/ml to 100 ng/ml, 2 ng/ml to 50 ng/ml, 5 ng/ml to 200 ng/ml, 10 ng/ml to 200 ng/ml, 15 ng/ml to 200 ng/ml, 5 ng/ml to 150 ng/ml, 10 ng/ml to 100 ng/ml, or 15 ng/ml to 50 ng/ml.

In an embodiment, the concentration of IL3 in the first culture medium composition may be in a range of 2 ng/ml to 200 ng/ml, 2 ng/ml to 150 ng/ml, 2 ng/ml to 100 ng/ml, 2 ng/ml to 50 ng/ml, 5 ng/ml to 200 ng/ml, 10 ng/ml to 200 ng/ml, 15 ng/ml to 200 ng/ml, 5 ng/ml to 150 ng/ml, 10 ng/ml to 100 ng/ml, or 15 ng/ml to 50 ng/ml.

In an embodiment, the concentration of VPA in the first culture medium composition may be in a range of 0.1 mM to 50 mM, 0.1 mM to 30 mM, 0.1 mM to 10 mM, 0.1 mM to 5 mM, 0.1 mM to 2 mM, 0.3 mM to 50 mM, 0.5 mM to 30 mM, 0.7 mM to 10 mM, 0.9 mM to 5 mM, 0.7 mM to 5 mM, or 0.5 mM to 3 mM.

In an embodiment, the concentration of SCF in the second culture medium composition may be in a range of 2 ng/ml to 200 ng/ml, 2 ng/ml to 150 ng/ml, 2 ng/ml to 100 ng/ml, 2 ng/ml to 50 ng/ml, 5 ng/ml to 200 ng/ml, 10 ng/ml to 200 ng/ml, 15 ng/ml to 200 ng/ml, 5 ng/ml to 150 ng/ml, 10 ng/ml to 100 ng/ml, or 15 ng/ml to 50 ng/ml.

In an embodiment, the concentration of TPO in the second culture medium composition may be in a range of 5 ng/ml to 500 ng/ml, 5 ng/ml to 400 ng/ml, 5 ng/ml to 300 ng/ml, 5 ng/ml to 200 ng/ml, 5 ng/ml to 100 ng/ml, 10 ng/ml to 500 ng/ml, 20 ng/ml to 500 ng/ml, 30 ng/ml to 500 ng/ml, 40 ng/ml to 500 ng/ml, 10 ng/ml to 400 ng/ml, 20 ng/ml to 300 ng/ml, 30 ng/ml to 200 ng/ml, or 40 ng/ml to 100 ng/ml.

In an embodiment, the concentration of PVA in the second culture medium composition may be in a range of 0.01 %(w/v) to 1 %(w/v), 0.01 %(w/v) to 0.8 %(w/v), 0.01 %(w/v) to 0.6 %(w/v), 0.01 %(w/v) to 0.4 %(w/v), 0.01 %(w/v) to 0.2 %(w/v), 0.01 %(w/v) to 0.1 %(w/v), 0.03 %(w/v) to 1 %(w/v), 0.05 %(w/v) to 1 %(w/v), 0.07 %(w/v) to 1 %(w/v), 0.09 %(w/v) to 1 %(w/v), 0.03 %(w/v) to 0.8 %(w/v), 0.05 %(w/v) to 0.6 %(w/v), 0.07 %(w/v) to 0.4 %(w/v), or 0.09 %(w/v) to 0.2 %(w/v).

In an embodiment, the concentration of bFGF in the second culture medium composition may be in a range of 1 ng/ml to 100 ng/ml, 1 ng/ml to 90 ng/ml, 1 ng/ml to 80 ng/ml, 1 ng/ml to 70 ng/ml, 1 ng/ml to 60 ng/ml, 1 ng/ml to 50 ng/ml, 1 ng/ml to 40 ng/ml, 1 ng/ml to 30 ng/ml, 1 ng/ml to 20 ng/ml, 3 ng/ml to 100 ng/ml, 5 ng/ml to 100 ng/ml, 7 ng/ml to 100 ng/ml, 9 ng/ml to 100 ng/ml, 3 ng/ml to 90 ng/ml, 5 ng/ml to 80 ng/ml, 7 ng/ml to 70 ng/ml, 9 ng/ml to 60 ng/ml, 15 ng/ml to 50 ng/ml, 17 ng/ml to 30 ng/ml or 1 ng/ml to 50 ng/ml.

In an embodiment, the concentration of butyzamide in the second culture medium composition may be in a range of 0.01 µM to 1 µM, 0.01 µM to 0.8 µM, 0.01 µM to 0.6 µM, 0.01 µM to 0.4 µM, 0.01 µM to 0.2 µM, 0.01 µM to 0.1 µM, 0.03 µM to 1 µM, 0.05 µM to 1 µM, 0.07 µM to 1 µM, 0.09 µM to 1 µM, 0.03 µM to 0.8 µM, 0.05 µM to 0.6 µM, 0.07 µM to 0.4 µM, or 0.09 µM to 0.2 µM.

In an embodiment, the concentration of IL6 in the second culture medium composition may be in a range of 2 ng/ml to 200 ng/ml, 2 ng/ml to 150 ng/ml, 2 ng/ml to 100 ng/ml, 2 ng/ml to 50 ng/ml, 5 ng/ml to 200 ng/ml, 10 ng/ml to 200 ng/ml, 15 ng/ml to 200 ng/ml, 5 ng/ml to 150 ng/ml, 10 ng/ml to 100 ng/ml, or 15 ng/ml to 50 ng/ml.

In an embodiment, the concentration of UM729 in the second culture medium composition may be in a range of 5 nM to 500 nM, 5 nM to 400 nM, 5 nM to 300 nM, 5 nM to 200 nM, 5 nM to 100 nM, 10 nM to 500 nM, 30 nM to 500 nM, 50 nM to 500 nM, 60 nM to 500 nM, 10 nM to 400 nM, 30 nM to 300 nM, 50 nM to 200 nM, or 60 nM to 100 nM.

In an embodiment, the concentration of M-CSF in the second culture medium composition may be in a range of 1 µM to 100 µM, 1 µM to 80 µM, 1 µM to 60 µM, 1 µM to 40 µM, 3 µM to 100 µM, 5 µM to 100 µM, 7 µM to 100 µM, 9 µM to 100 µM, 3 µM to 80 µM, 5 µM to 60 µM, 7 µM to 40 µM, or 9 µM to 20 µM.

In an embodiment, the concentration of SCF in the third culture medium composition may be in a range of 2 ng/ml to 200 ng/ml, 2 ng/ml to 150 ng/ml, 2 ng/ml to 100 ng/ml, 2 ng/ml to 50 ng/ml, 5 ng/ml to 200 ng/ml, 10 ng/ml to 200 ng/ml, 15 ng/ml to 200 ng/ml, 5 ng/ml to 150 ng/ml, 10 ng/ml to 100 ng/ml, or 15 ng/ml to 50 ng/ml.

In an embodiment, the concentration of TPO in the third culture medium composition may be in a range of 5 ng/ml to 500 ng/ml, 5 ng/ml to 400 ng/ml, 5 ng/ml to 300 ng/ml, 5 ng/ml to 200 ng/ml, 5 ng/ml to 100 ng/ml, 10 ng/ml to 500 ng/ml, 20 ng/ml to 500 ng/ml, 30 ng/ml to 500 ng/ml, 40 ng/ml to 500 ng/ml, 10 ng/ml to 400 ng/ml, 20 ng/ml to 300 ng/ml, 30 ng/ml to 200 ng/ml, or 40 ng/ml to 100 ng/ml.

In an embodiment, the concentration of IL6 in the third culture medium composition may be in a range of 2 ng/ml to 200 ng/ml, 2 ng/ml to 150 ng/ml, 2 ng/ml to 100 ng/ml, 2 ng/ml to 50 ng/ml, 5 ng/ml to 200 ng/ml, 10 ng/ml to 200 ng/ml, 15 ng/ml to 200 ng/ml, 5 ng/ml to 150 ng/ml, 10 ng/ml to 100 ng/ml, or 15 ng/ml to 50 ng/ml.

In an embodiment, the concentration of fasudil in the third culture medium composition may be in a range of 1 µM to 100 µM, 1 µM to 80 µM, 1 µM to 60 µM, 1 µM to 40 µM, 1 µM to 20 µM, 3 µM to 100 µM, 5 µM to 100 µM, 7 µM to 100 µM, 9 µM to 100 µM, 3 µM to 80 µM, 5 µM to 60 µM, 7 µM to 40 µM, or 9 µM to 20 µM.

In an embodiment, the concentration of butyzamide in the third culture medium composition may be in a range of 0.01 µM to 1 µM, 0.01 µM to 0.8 µM, 0.01 µM to 0.6 µM, 0.01 µM to 0.4 µM, 0.01 µM to 0.2 µM, 0.01 µM to 0.1 µM, 0.02 µM to 1 µM, 0.03 µM to 1 µM, 0.04 µM to 1 µM, 0.05 µM to 1 µM, 0.02 µM to 0.8 µM, 0.03 µM to 0.6 µM, 0.04 µM to 0.4 µM, or 0.05 µM to 0.2 µM.

In an embodiment, the concentration of bFGF in the third culture medium composition may be in a range of 1 ng/ml to 100 ng/ml, 1 ng/ml to 90 ng/ml, 1 ng/ml to 80 ng/ml, 1 ng/ml to 70 ng/ml, 1 ng/ml to 60 ng/ml, 1 ng/ml to 50 ng/ml, 1 ng/ml to 40 ng/ml, 1 ng/ml to 30 ng/ml, 1 ng/ml to 20 ng/ml, 3 ng/ml to 100 ng/ml, 5 ng/ml to 100 ng/ml, 7 ng/ml to 100 ng/ml, 9 ng/ml to 100 ng/ml, 3 ng/ml to 90 ng/ml, 5 ng/ml to 80 ng/ml, 7 ng/ml to 70 ng/ml, 9 ng/ml to 60 ng/ml, 15 ng/ml to 50 ng/ml, 17 ng/ml to 30 ng/ml, or 1 ng/ml to 50 ng/ml.

In the present specification, "positive or +" with respect to a cell marker may mean that the marker is present in a greater amount or at a higher concentration compared to another cell that serves as a reference. A cell may be positive for a marker if that marker is present inside or on the surface of the cell, thereby allowing the cell to be distinguished from one or more other cell types using that marker. This term can also mean that the cell possesses the marker in an amount sufficient to produce a signal-for example, a signal from a cell measurement device-that is greater than a background value. For example, a cell may be detectably labeled with an antibody specific for CD56, and if the signal from this antibody is detectably greater than a control (e.g., a background value), the cell may be represented as "positive for CD56" or "CD56+". The term "negative or -" may mean that the marker cannot be detected relative to a background value, even when using an antibody specific for that particular cell surface marker. For example, if a cell cannot be detectably labeled with an antibody specific for CD3, the cell may be represented as "negative for CD3" or "CD3⁻".

In an embodiment, in the platelet progenitor cells differentiated by the culture medium composition for inducing differentiation of stem cells into platelet progenitor cells of the present disclosure, 50 %, 60 %, 70 %, 80 %, or 90 % or more of the cells in the total cell population may be positive for CD41a and CD42b.

In an embodiment, the stem cells may be pluripotent stem cells.

In an embodiment, the stem cells may be human embryonic stem cells (hESCs) or human induced pluripotent stem cells (hiPSCs).

As used herein, the term "stem cell" refers to a cell having self-renewal ability and the ability to differentiate into two or more cells, and can be classified into totipotent stem cells, pluripotent stem cells, and multipotent stem cells.

In the present disclosure, 'human pluripotent stem cells (hPSCs)' refers to stem cells having pluripotent or totipotent self-renewal ability to differentiate into cells of all tissues of an individual, and may include embryonic stem cells or induced pluripotent stem cells. 'Embryonic stem cells' are derived from the inner cell mass extracted from a blastocyst-stage embryo just before implantation in the mother's uterus and cultured in vitro, possessing pluripotent or totipotent self-renewal ability to differentiate into cells of all tissues of an individual. In a broad sense, this term also encompasses embryoid bodies derived from embryonic stem cells. 'Induced pluripotent stem cells' refers to cells that have been induced to have pluripotent differentiation potential from differentiated cells through an artificial reprogramming process, and are also called reprogrammed stem cells. The artificial reprogramming process can be performed by introducing reprogramming factors in a viral-mediated manner using retroviruses or lentiviruses, or in a nonviral-mediated manner using proteins and cell extracts, and may also include a reprogramming process involving stem cell extracts, chemical compounds, or the like. An induced pluripotent stem cell has properties almost identical to those of an embryonic stem cell, specifically exhibiting a similar cell morphology and similar gene/protein expression patterns, possessing pluripotency in vitro and in vivo, forming a teratoma, and, when inserted into a mouse blastocyst, forming a chimera mouse and enabling germline transmission of genes.

Another aspect provides a method of inducing differentiation of a stem cell into a platelet progenitor cell, the method including: (i) inducing differentiation of a stem cell into a hematopoietic progenitor cell; (ii) inducing differentiation of the hematopoietic progenitor cell into a megakaryocyte progenitor cell; and (iii) inducing maturation of the megakaryocyte progenitor cell into a platelet progenitor cell. The same parts described above also apply equally to this method.

Step (i) will be described in detail.

Step (i) may include inducing differentiation of stem cells into hematopoietic stem cells or hematopoietic progenitor cells in the presence of a first culture medium composition.

In an embodiment, step (i) may include inducing mesoderm from stem cells; inducing hemangioblast; including endothelial-to-hematopoietic transition; and/or inducing differentiation into hematopoietic stem cells or hematopoietic progenitor cells.

In an embodiment, step (i) may be performed for a duration of 1 day to 20 days, 2 days to 19 days, 3 days to 18 days, 4 days to 17 days, or 5 days to 16 days.

During the differentiation induction in step (i), the first culture medium composition may be replaced with a first culture medium composition having a different composition.

In an embodiment, the step of inducing mesoderm may be performed in the presence of a medium including CHIR.

In an embodiment, the step of inducing hemangioblast may be performed in the presence of a medium including BMP4, VEGF, and/or bFGF.

In an embodiment, the step including endothelial-to-hematopoietic transition may be performed in the presence of a medium including VEGF, bFGF, SB-431542, and/or retinoic acid.

In an embodiment, the step of inducing differentiation into hematopoietic stem cells or hematopoietic progenitor cells may be performed in the presence of PVA, bFGF, SCF, IL3, and/or VPA.

Step (ii) will be described in detail.

Step (ii) may include inducing differentiation of hematopoietic progenitor cells or hematopoietic stem cells into megakaryocyte progenitor cells in the presence of a second culture medium composition.

In an embodiment, step (ii) may be performed for a duration of 1 day to 20 days, 5 days to 18 days, or 7 days to 16 days.

During the differentiation induction in step (ii), the first culture medium composition may be replaced with a second culture medium composition having a different composition.

In an embodiment, the step of inducing differentiation of hematopoietic progenitor cells or hematopoietic stem cells into megakaryocyte progenitor cells may be performed in the presence of a medium including SCF, TPO, PVA, bFGF, butyzamide, IL6, and/or UM729.

Step (iii) will be described in detail.

Step (iii) may include inducing maturation of megakaryocyte progenitor cells into platelet progenitor cells in the presence of a third culture medium composition.

In an embodiment, step (iii) may be performed for a duration of 5 days to 14 days, or 7 days to 12 days.

During the differentiation induction in step (iii), the third culture medium composition may be replaced with a third culture medium composition having a different composition.

In an embodiment, the step of inducing maturation of megakaryocyte progenitor cells into platelet progenitor cells may be performed in the presence of a medium including SCF, TPO, IL6, fasudil, bFGF, and/or butyzamide.

In an embodiment, the method may include: (i) inducing differentiation of stem cells into hematopoietic progenitor cells or hematopoietic stem cells in the presence of a first culture medium composition including at least one selected from the group consisting of CHIR99021, Bone Morphogenetic Protein 4 (BMP4), Vascular Endothelial Growth Factor (VEGF), Basic Fibroblast Growth Factor (bFGF), retinoic acid, SB-431542, polyvinyl alcohol (PVA), Stem Cell Factor (SCF), Interleukin 3 (IL3), valproic acid (VPA), and combinations thereof; (ii) inducing differentiation of hematopoietic progenitor cells or hematopoietic stem cells into megakaryocyte progenitor cells in the presence of a second culture medium composition including at least one selected from the group consisting of SCF, Thrombopoietin (TPO), PVA, bFGF, butyzamide, Interleukin 6 (IL6), UM729, Macrophage Colony-Stimulating Factor (M-CSF), and combinations thereof; and (iii) inducing maturation of megakaryocyte progenitor cells into platelet progenitor cells in the presence of a third culture medium composition including at least one selected from the group consisting of SCF, TPO, Interleukin 6 (IL6), fasudil, butyzamide, bFGF, and combinations thereof.

In an embodiment, platelets derived from platelet progenitor cells differentiated by the aforementioned method of inducing differentiation of stem cells into platelet progenitor cells are provided.

Platelets derived from the platelet progenitor cells can be obtained by culturing the platelet progenitor cells-which are obtained by the aforementioned method of inducing differentiation of stem cells into platelet progenitor cells-and then harvesting the platelets from the resulting culture.

The harvesting of the platelets may be induced and achieved in the presence of a medium including bFGF, butyzamide, fasudil, and/or KP-457.

In an embodiment, a platelet preparation or a blood preparation including the platelets is provided.

The production of the platelet preparation according to the present disclosure may include a process of culturing megakaryocytes by the method described herein to produce platelets and harvesting a platelet-rich fraction from the culture, and a process of removing blood cell components other than platelets from the platelet fraction. The process of removing blood cell components may be performed, for example, by using a leukocyte removal filter to eliminate blood cell components other than platelets. A more specific method of producing a platelet preparation is described, for example, in International Publication No. WO 2011/034073.

The production of the blood preparation according to the present disclosure may include a process of producing a platelet preparation by the method described herein, and a process of mixing the platelet preparation with other components. Examples of other components include red blood cells.

Other components that contribute to cell stabilization may be added to the platelet preparation and the blood preparation.

Another aspect provides a kit for inducing differentiation of stem cells into platelet progenitor cells, the kit including: (i) a first culture medium composition for inducing differentiation of stem cells into hematopoietic progenitor cells or hematopoietic stem cells, including at least one selected from the group consisting of CHIR99021, Bone Morphogenetic Protein 4 (BMP4), Vascular Endothelial Growth Factor (VEGF), Basic Fibroblast Growth Factor (bFGF), retinoic acid, SB-431542, polyvinyl alcohol (PVA), Stem Cell Factor (SCF), Interleukin 3 (IL3), valproic acid (VPA), and combinations thereof; (ii) a second culture medium composition for inducing differentiation of hematopoietic progenitor cells or hematopoietic stem cells into megakaryocyte progenitor cells, including at least one selected from the group consisting of SCF, Thrombopoietin (TPO), PVA, bFGF, butyzamide, Interleukin 6 (IL6), UM729, Macrophage Colony-Stimulating Factor (M-CSF), and combinations thereof; and (iii) a third culture medium composition for maturation of megakaryocyte progenitor cells into platelet progenitor cells, including at least one selected from the group consisting of SCF, TPO, Interleukin 6 (IL6), fasudil, butyzamide, bFGF, and combinations thereof.

### Advantageous Effects of the Invention

According to one aspect, the culture medium composition for inducing differentiation of stem cells into platelet progenitor cells can enhance the differentiation efficiency into platelet progenitor cells and has the effect of differentiating into platelet progenitor cells without genetic manipulation.

### Brief Description of Drawings

FIGS. 1 and 2 show schematic diagrams illustrating the process of differentiating a stem cell into a platelet progenitor cell.
FIGS. 3 and 4 show results confirming CD markers specific to the platelet progenitor cells after induction of differentiation from a stem cell.
FIG. 5 compares the efficiency of differentiation into platelet progenitor cells, as determined by CD markers, between a conventional induction method and the platelet progenitor cells obtained in Example 1 of the present disclosure.
FIGS. 6 and 7 show morphological characteristics of the platelet progenitor cells differentiated according to the present disclosure.
FIGS. 8 and 9 show results confirming whether the platelet progenitor cells differentiated according to the present disclosure release platelets.

### Mode for the Invention

Hereinafter, the present disclosure will be described in more detail through Examples. However, these Examples are intended to illustrate the present disclosure, and the scope of the present disclosure is not limited thereto.

Terms or words used in the specification and claims of the present disclosure should not be interpreted as being limited to ordinary or dictionary meanings, and should be interpreted with meanings and concepts consistent with the technical spirit of the present disclosure based on the principle that the inventor can appropriately define the concept of terms to describe his or her own invention in the best way possible.

Throughout the specification of the present disclosure, when a part is stated to "comprise" a certain component, this means that other components may be further included, rather than excluding other components, unless specifically stated otherwise.

Throughout the specification of the present disclosure, "A and/or B" means A or B, or A and B.

### Example 1.

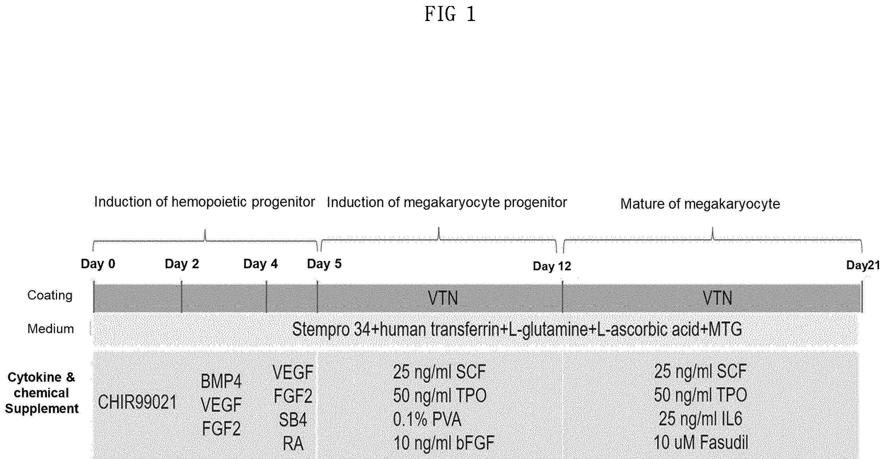

A schematic diagram of the process of differentiating stem cells into platelet progenitor cells (megakaryocytes) according to Example 1 is shown in FIG. 1.

### 1.1. Differentiation into Hematopoietic Progenitor Cells

Human pluripotent stem cells (hPSCs) were maintained in stemMACS-iPS brew media for 2 days. The basal differentiation medium was Stempro34-SFM (+stempro sup) + 200 µg/ml human Transferrin + 2 mM L-glutamine + 0.5 mM L-Ascorbic acid + 0.45 mM MTG (1-thioglycerol) + 1 % penicillin/streptomycin.

CHIR99021, known to induce mesoderm, was treated alone at a concentration of 5 µM for 2 days. Additionally, cells were treated for 2 days in a differentiation culture medium supplemented with BMP4 50 ng/ml, VEGF 50 ng/ml, and bFGF 100 ng/ml. Thereafter, for the next day, cells were cultured in a culture medium supplemented with VEGF 50 ng/ml, bFGF 50 ng/ml, SB-431542 10 µM, and retinoic acid 1 µM.

### 1.2. Differentiation into Megakaryocyte Progenitor Cells

After differentiation into hematopoietic progenitor cells, from Day 5 to Day 12, cells were cultured in a culture medium supplemented with PVA 0.1 %, SCF 25 ng/ml, TPO 50 ng/ml, and bFGF 10 ng/ml, with daily medium changes.

### 1.3. Maturation into Platelet Progenitor Cells (Megakaryocytes)

After differentiation into megakaryocyte progenitor cells, from Day 12 to Day 21, cells were cultured with the addition of SCF 25 ng/ml, TPO 50 ng/ml, IL6 25 ng/ml, and fasudil 10 µM.

### Example 2.

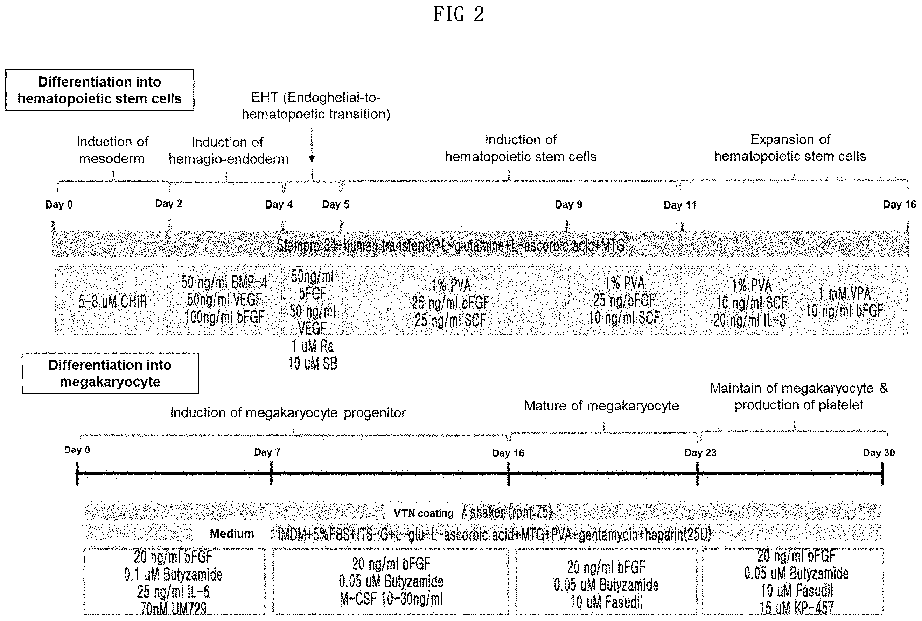

A schematic diagram of the process of differentiating stem cells into platelet progenitor cells (megakaryocytes) according to Example 2 is shown in FIG. 2.

### 2.1. Differentiation into Hematopoietic Stem Cells

Human pluripotent stem cells (hPSCs) were maintained in stemMACS-iPS brew media for 2 days. The basal differentiation medium was Stempro34-SFM (+stempro sup) + 200 µg/ml human Transferrin + 2 mM L-glutamine + 0.5 mM L-Ascorbic acid + 0.45 mM MTG (1-thioglycerol) + 1 % penicillin/streptomycin.

CHIR99021, known to induce mesoderm, was treated alone at a concentration of 5 µM to 8 µM for 2 days. Additionally, cells were treated for 2 days in a differentiation culture medium supplemented with BMP4 50 ng/ml, VEGF 50 ng/ml, and bFGF 100 ng/ml to induce hemangioblast. Thereafter, for the next day, cells were cultured in a culture medium supplemented with VEGF 50 ng/ml, bFGF 50 ng/ml, SB-431542 10 µM, and retinoic acid 1 µM to induce endothelial-to-hematopoietic transition (EHT).

From day 5 to day 9 after differentiation, cells were cultured for 4 days in a culture medium supplemented with PVA 1 %, bFGF 10 ng/ml, and SCF 25 ng/ml, and from Day 9 to Day 11, cells were cultured for 2 days in a culture medium supplemented with PVA 1 %, bFGF 10 ng/ml, and SCF 10 ng/ml. Additionally, from Day 11 to Day 16 after differentiation, cells were cultured in a culture medium supplemented with PVA 1 %, bFGF 10 ng/ml, SCF 10 ng/ml, IL3 20 ng/ml, and VPA 1 mM to differentiate into hematopoietic stem cells.

### 2.2. Differentiation into Megakaryocyte Progenitor Cells

The hematopoietic stem cells differentiated according to Example 2.1. were differentiated into megakaryocyte progenitor cells. The basal differentiation medium was IMDM + 5 % FBS + 1 % ITS-X + 4 mM L-Glutamine + 0.5 mM L-Ascorbic Acid + 0.45 mM MTG + 1 % PVA + 50 µg/ml gentamycin + 25U heparin.

After differentiation into hematopoietic stem cells, for 7 days, cells were cultured in a culture medium supplemented with bFGF 20 ng/ml, butyzamide 0.1 µM, IL6 25 ng/ml, and UM729 70 nM, and from Day 7 to Day 16 after differentiation, for 9 days, cells were cultured in a culture medium supplemented with bFGF 20 ng/ml, butyzamide 0.05 µM, and M-CSF 10 to 30 ng/ml.

### 2.3. Maturation into Platelet Progenitor Cells (Megakaryocytes)

After differentiation into megakaryocyte progenitor cells, from Day 16 to Day 23, cells were cultured in a culture medium supplemented with bFGF 20 ng/ml, butyzamide 0.05 µM, and fasudil 10 µM to mature the megakaryocytes.

### Comparative Example 1.

### 1.1. Differentiation into Hematopoietic Progenitor Cells

Human pluripotent stem cells (hPSCs) were cultured for 6 days using STEMSpan-ACF media + 50 ng/ml BMP4 + 50 ng/ml VEGF + 50 ng/ml bFGF.

### 1.2. Differentiation into Megakaryocyte Progenitor Cells

After differentiation into hematopoietic progenitor cells, from Day 6 to Day 12, differentiation was induced using STEMdiff APEL media + 25 ng/ml TPO + 25 ng/ml SCF + 25 ng/ml FLT3L + 10 ng/ml IL3 + 10 ng/ml IL6 + 5 U/ml heparin.

### 1.3. Megakaryocyte Maturation and Platelet Production

After differentiation into megakaryocyte progenitor cells, from Day 12 to Day 20, cells were cultured with the addition of STEMSpan-ACF + 25 ng/ml TPO + 25 ng/ml SCF + 10 ng/ml IL6 + 10 ng/ml IL9 + 5 U/ml heparin.

### Comparative Example 2.

### 2.1. Differentiation into Hematopoietic Progenitor Cells

Human pluripotent stem cells (hPSCs) were maintained in stemMACS-iPS brew media for 2 days. The basal differentiation medium was Stempro34-SFM (+stempro sup) + 200 µg/ml human Transferrin + 2 mM L-glutamine + 0.5 mM L-Ascorbic acid + 0.45 mM MTG (1-thioglycerol) + 1 % penicillin/streptomycin.

CHIR99021, known to induce mesoderm, was treated alone at a concentration of 5 µM for 2 days. Additionally, cells were treated for 2 days in a differentiation culture medium supplemented with BMP4 50 ng/ml, VEGF 50 ng/ml, and bFGF 100 ng/ml. Thereafter, for the next day, cells were cultured in a culture medium supplemented with VEGF 50 ng/ml, bFGF 50 ng/ml, SB-431542 10 µM, and retinoic acid 1 µM.

### 2.2. Differentiation into Megakaryocyte Progenitor Cells

After differentiation into hematopoietic progenitor cells, from Day 5 to Day 12, cells were cultured in a culture medium supplemented with PVA 0.1 %, SCF 25 ng/ml, TPO 50 ng/ml, and bFGF 10 ng/ml, with daily medium changes.

### 2.3. Maturation into Platelet Progenitor Cells (Megakaryocytes)

After differentiation into megakaryocyte progenitor cells, from Day 12 to Day 21, cells were cultured with the addition of SCF 25 ng/ml, TPO 50 ng/ml, IL6 25 ng/ml, and SR1 1 µM.

### Experimental Example 1: Confirmation of Platelet Progenitor Cell-Specific Markers

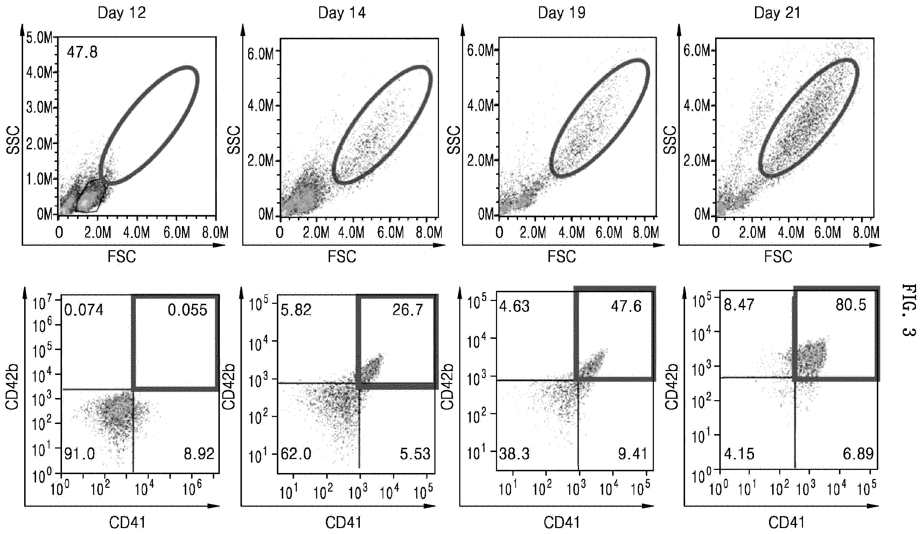

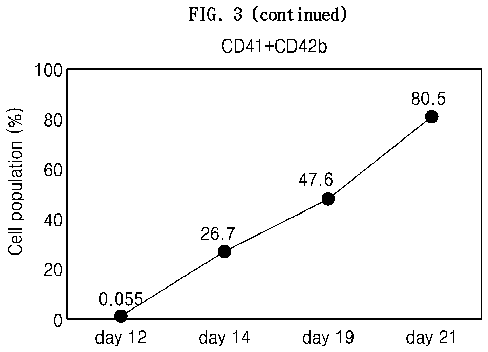

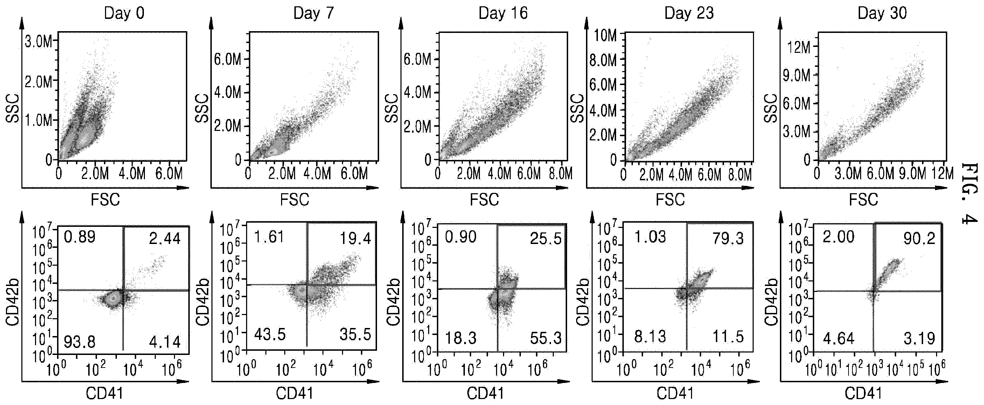

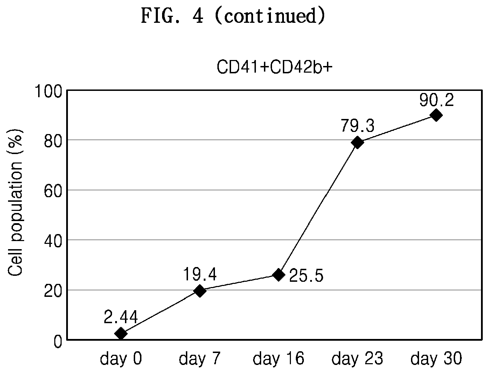

To confirm whether the cells obtained in Examples 1 and 2 were differentiated into platelet progenitor cells, CD markers specific to platelet progenitor cells were confirmed using FACS (Fluorescence-activated cell sorting, BD FACSCalibur^{™}). Specifically, cells were harvested at different differentiation time points and CD markers were analyzed, and the results are shown in FIG. 3 and FIG. 4.

As shown in FIG. 3, CD41a and CD42b, which are megakaryocyte markers and were hardly expressed on Day 12 of differentiation, were expressed at 26.7 % on Day 14, 47.6 % on Day 19, and 80.5 % on Day 21, confirming that differentiation proceeded with increasing efficiency.

As shown in FIG. 4, CD41a and CD42b, which are megakaryocyte markers, were expressed at 19.4 % on Day 7 of differentiation, 25.5 % on Day 16, 79.3 % on Day 23, and 90.2 % on Day 30, confirming that differentiation proceeded with increasing efficiency.

From these results, it was found that human pluripotent stem cells can be efficiently differentiated into platelet progenitor cells through the methods of Examples 1 and 2.

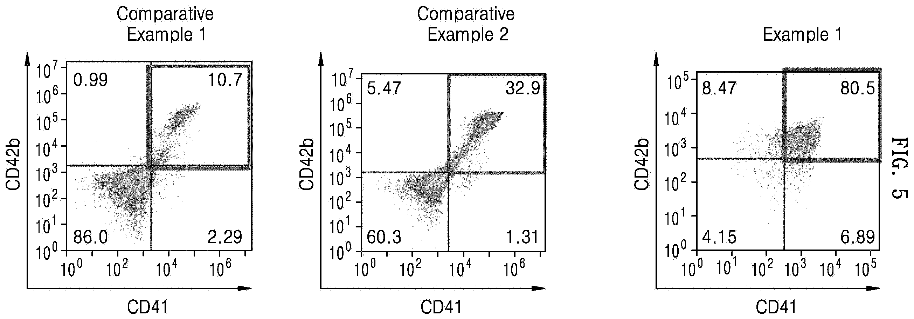

In addition, to investigate the differentiation efficiency, platelet progenitor cells obtained in Comparative Examples were confirmed for CD markers in the same manner and compared. The results are shown in FIG. 5.

As shown in FIG. 5, when differentiation was performed by the method of Comparative Example 1, the number of CD41a/CD42b double positive cells was 10.7 %, and when differentiation was performed by the method of Comparative Example 2 with the addition of SR1, it was 32.9 %. It was found that when differentiation was performed by the method of Example 1, high differentiation efficiency (80.5 %) was shown with the addition of fasudil.

### Experimental Example 2: Confirmation of Morphology of Differentiated Platelet Progenitor Cells

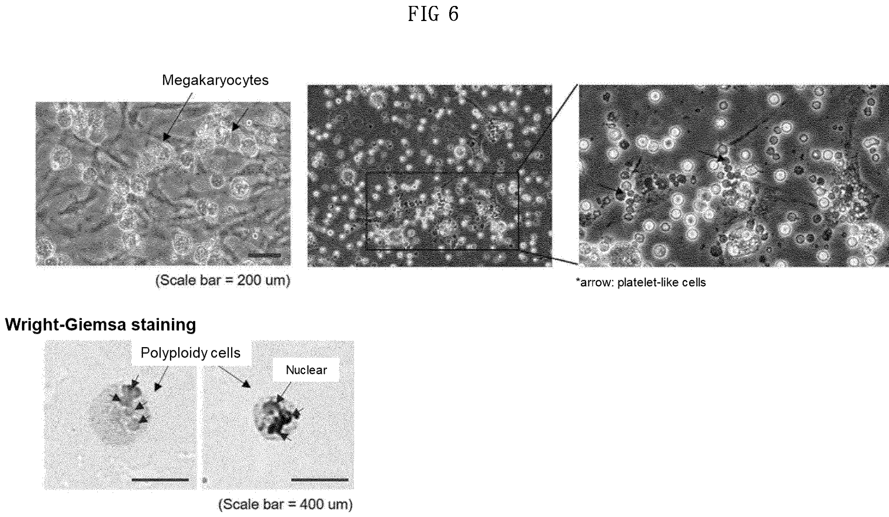

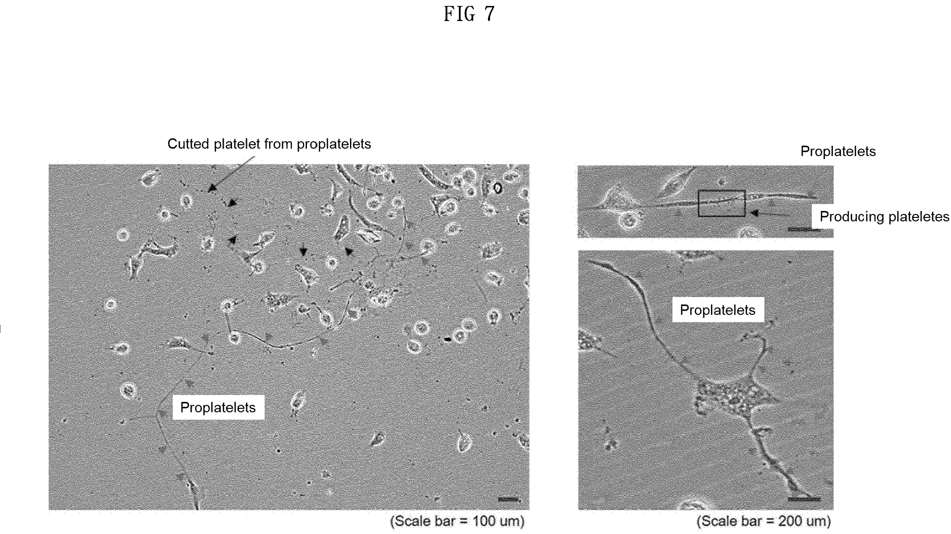

To investigate the morphological characteristics of the cells obtained in Examples 1 and 2, the differentiated platelet progenitor cells were observed under a microscope and by Giemsa staining. The results are shown in FIGS. 6 and 7.

As shown in FIGS. 6 and 7, it was found that the differentiated platelet progenitor cells showed a multinucleated structure.

### Experimental Example 3: Confirmation of Platelet Release

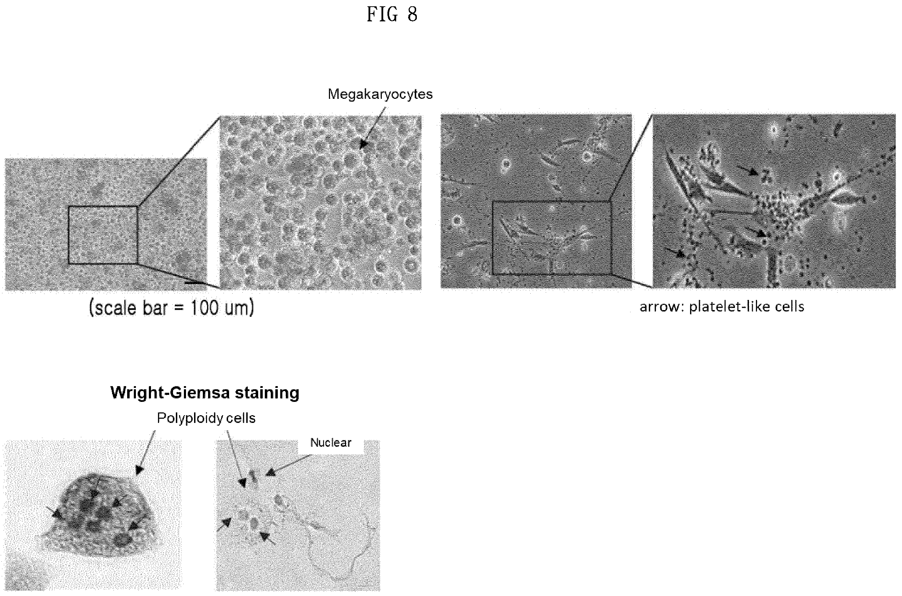

To investigate whether the cells obtained in Example 1 released platelets, on Day 23 after differentiation, the cells were transferred to another plate coated with gelatin, and the platelet formation process was observed while culturing in the medium of Example 1.3. The results are shown in FIG. 8.

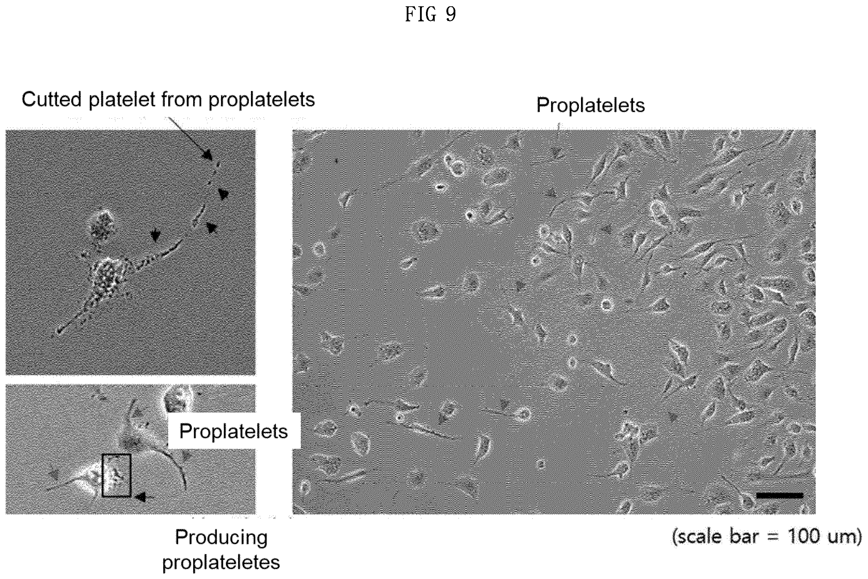

Additionally, to investigate whether the cells obtained in Example 2 produced platelets, from Day 23 after differentiation, cells were cultured for 7 days in a culture medium supplemented with bFGF 20 ng/ml, butyzamide 0.05 µM, fasudil 10 µM, and KP-457 15 µM, and the platelet formation process was observed. The results are shown in FIG. 9.

As shown in FIGS. 8 and 9, it was confirmed that on Day 28 of differentiation (7 days after transfer to another plate), the platelet progenitor cells adhered to the culture dish and released platelets.

From these results, it was found that platelets can be obtained by culturing the cells obtained in Examples 1 and 2.

## Claims

1. A culture medium composition for inducing differentiation of a stem cell into a platelet progenitor cell, the culture medium composition comprising:
(i) a first culture medium composition for inducing differentiation of the stem cell into a hematopoietic progenitor cell or a hematopoietic stem cell, the first culture medium composition comprising at least one selected from the group consisting of CHIR99021, Bone Morphogenetic Protein 4 (BMP4), Vascular Endothelial Growth Factor (VEGF), Basic Fibroblast Growth Factor (bFGF), retinoic acid, SB-431542, polyvinyl alcohol (PVA), Stem Cell Factor (SCF), Interleukin 3 (IL3), valproic acid (VPA), and combinations thereof;
(ii) a second culture medium composition for inducing differentiation of the hematopoietic progenitor cell or the hematopoietic stem cell into a megakaryocyte progenitor cell, the second culture medium composition comprising at least one selected from the group consisting of SCF, Thrombopoietin (TPO), PVA, bFGF, butyzamide, Interleukin 6 (IL6), UM729, Macrophage Colony-Stimulating Factor (M-CSF), and combinations thereof; and
(iii) a third culture medium composition for inducing maturation of the megakaryocyte progenitor cell into a platelet progenitor cell, the third culture medium composition comprising at least one selected from the group consisting of SCF, TPO, Interleukin 6 (IL6), fasudil, butyzamide, bFGF, and combinations thereof.

2. The culture medium composition of claim 1, wherein, in the first culture medium composition, a concentration of the CHIR99021 is in a range of 0.5 µM to 50 µM, a concentration of the BMP4 is in a range of 50 ng/ml to 500 ng/ml, a concentration of the VEGF is in a range of 5 ng/ml to 500 ng/ml, a concentration of the bFGF is in a range of 1 ng/ml to 1,000 ng/ml, a concentration of the retinoic acid is in a range of 0.1 µM to 10 µM, and a concentration of the SB-431542 is in a range of 1 µM to 100 µM.

3. The culture medium composition of claim 1, wherein, in the first culture medium composition, a concentration of the CHIR99021 is in a range of 0.5 µM to 50 µM, a concentration of the BMP4 is in a range of 50 ng/ml to 500 ng/ml, a concentration of the VEGF is in a range of 5 ng/ml to 500 ng/ml, a concentration of the bFGF is in a range of 1 ng/ml to 1,000 ng/ml, a concentration of the retinoic acid is in a range of 0.1 µM to 10 µM, a concentration of the SB-431542 is in a range of 1 µM to 100 µM, a concentration of the PVA is in a range of 0.01 %(w/v) to 10 %(w/v), a concentration of the SCF is in a range of 2 ng/ml to 200 ng/ml, a concentration of the IL3 is in a range of 2 ng/ml to 200 ng/ml, and a concentration of the VPA is in a range of 0.1 mM to 50 mM.

4. The culture medium composition of claim 1, wherein, in the second culture medium composition, a concentration of the SCF is in a range of 2 ng/ml to 200 ng/ml, a concentration of the TPO is in a range of 5 ng/ml to 500 ng/ml, a concentration of the PVA is in a range of 0.01 %(w/v) to 1 %(w/v), and a concentration of the bFGF is in a range of 1 ng/ml to 100 ng/ml.

5. The culture medium composition of claim 1, wherein, in the second culture medium composition, a concentration of the bFGF is in a range of 1 ng/ml to 100 ng/ml, a concentration of the butyzamide is in a range of 0.01 µM to 1 µM, a concentration of the IL6 is in a range of 2 ng/ml to 200 ng/ml, a concentration of the UM729 is in a range of 5 nM to 500 nM, and a concentration of the M-CSF is in a range of 1 µM to 100 µM.

6. The culture medium composition of claim 1, wherein, in the third culture medium composition, a concentration of the SCF is in a range of 2 ng/ml to 200 ng/ml, a concentration of the TPO is in a range of 5 ng/ml to 500 ng/ml, a concentration of the IL6 is in a range of 2 ng/ml to 200 ng/ml, and a concentration of the fasudil is in a range of 1 µM to 100 µM.

7. The culture medium composition of claim 1, wherein, in the third culture medium composition, a concentration of the bFGF is in a range of 1 ng/ml to 100 ng/ml, a concentration of the butyzamide is in a range of 0.01 µM to 1 µM, and a concentration of the fasudil is in a range of 1 µM to 100 µM.

8. The culture medium composition of claim 1, wherein at least 50 % of a total cell population of differentiated platelet progenitor cells is positive for CD41a and CD42b.

9. The culture medium composition of claim 1, wherein the stem cell is a human embryonic stem cell (hESC) or a human induced pluripotent stem cell (hiPSC).

10. A method of inducing differentiation of a stem cell into a platelet progenitor cell, the method comprising:
(i) inducing differentiation of the stem cell into a hematopoietic progenitor cell or a hematopoietic stem cell in the presence of a first culture medium composition comprising at least one selected from the group consisting of CHIR99021, Bone Morphogenetic Protein 4 (BMP4), Vascular Endothelial Growth Factor (VEGF), Basic Fibroblast Growth Factor (bFGF), retinoic acid, SB-431542, polyvinyl alcohol (PVA), Stem Cell Factor (SCF), Interleukin 3 (IL3), valproic acid (VPA), and combinations thereof;
(ii) inducing differentiation of the hematopoietic progenitor cell or the hematopoietic stem cell into a megakaryocyte progenitor cell in the presence of a second culture medium composition comprising at least one selected from the group consisting of SCF, Thrombopoietin (TPO), PVA, bFGF, butyzamide, Interleukin 6 (IL6), UM729, Macrophage Colony-Stimulating Factor (M-CSF), and combinations thereof; and
(iii) inducing maturation of the megakaryocyte progenitor cell into a platelet progenitor cell in the presence of a third culture medium composition comprising at least one selected from the group consisting of SCF, TPO, Interleukin 6 (IL6), fasudil, butyzamide, bFGF, and combinations thereof.

11. The method of claim 10, wherein step (i) comprises culturing the stem cell in a medium composition comprising BMP4, VEGF, and bFGF for 1 day to 20 days.

12. The method of claim 10, wherein step (ii) comprises culturing the hematopoietic progenitor cell or the hematopoietic stem cell in a medium composition comprising bFGF or butyzamide for 1 day to 20 days.

13. The method of claim 10, wherein step (iii) comprises culturing the megakaryocyte progenitor cell in a medium composition comprising fasudil for 5 days to 14 days.

14. Platelets derived from a platelet progenitor cell differentiated by the method of claim 10.

15. A platelet preparation or a blood preparation, comprising the platelets of claim 14.

16. A kit for inducing differentiation of a stem cell into a platelet progenitor cell, the kit comprising:
(i) a first culture medium composition for inducing differentiation of the stem cell into a hematopoietic progenitor cell or a hematopoietic stem cell, the first culture medium composition comprising at least one selected from the group consisting of CHIR99021, Bone Morphogenetic Protein 4 (BMP4), Vascular Endothelial Growth Factor (VEGF), Basic Fibroblast Growth Factor (bFGF), retinoic acid, SB-431542, polyvinyl alcohol (PVA), Stem Cell Factor (SCF), Interleukin 3 (IL3), valproic acid (VPA), and combinations thereof;
(ii) a second culture medium composition for inducing differentiation of the hematopoietic progenitor cell or the hematopoietic stem cell into a megakaryocyte progenitor cell, the second culture medium composition comprising at least one selected from the group consisting of SCF, Thrombopoietin (TPO), PVA, bFGF, butyzamide, Interleukin 6 (IL6), UM729, Macrophage Colony-Stimulating Factor (M-CSF), and combinations thereof; and
(iii) a third culture medium composition for inducing maturation of the megakaryocyte progenitor cell into a platelet progenitor cell, the third culture medium composition comprising at least one selected from the group consisting of SCF, TPO, Interleukin 6 (IL6), fasudil, butyzamide, bFGF, and combinations thereof.
